# EUROPÄISCHE PATENTSCHRIFT

(11) **EP 0 782 866 B1**
(45) Veröffentlichungstag und Bekanntmachung des Hinweises auf die Patenterteilung: **24.04.2002**
(21) Anmeldenummer: 96119492.5
(22) Anmeldetag: 05.12.1996
(51) Int. Cl.: A61M 11/00, B05B 11/00, B05B 1/34

(54) **Austragkopf für Medien, insbesondere zur medikamentösen Behandlung des Rachens**
Dispenser head, especially for drug treatment of the throat
Tête de diffuseur, en particulier pour le traitement médicamenteux de la gorge

(30) Priorität: 04.01.1996 DE 19600123
(43) Veröffentlichungstag der Anmeldung: 09.07.1997
(73) Patentinhaber: Ing. Erich Pfeiffer GmbH, 78315 Radolfzell (DE)
(72) Erfinder: Ritsche, Stefan, 78315 Radolfzell (DE); Amann, Esther, 78315 Radolfzell (DE)
(74) Vertreter: Patentanwälte Ruff, Wilhelm, Beier, Dauster & Partner

(56) Entgegenhaltungen:
- FR-A- 2 070 630
- US-A- 2 974 880
- US-A- 3 990 639
- US-A- 4 801 093
- US-A- 5 121 883

## Beschreibung

Die Erfindung betrifft einen Austragkopf nach dem Oberbegriff des Patentanspruches 1. Mit dem Austragkopf können flüssige, pastöse, pulverförmige oder ähnliche fließfähige Medien durch einen Düsenauslaß, insbesondere in einem Sprühkegel o. dgl. feinst zerstäubt, ausgebracht werden. Bevorzugt soll der Düsenauslaß tief, beispielsweise mindestens 20, 40 oder 50 mm tief in eine Körperöffnung, wie die Mundhöhle, eingeführt und dann das Medium ausgetragen werden können.

Der Austragkopf weist einen im wesentlichen seine gesamten Außenflächen bildenden Kopfkörper bzw. einen in einer Mittelachse liegenden Kopfteil auf, welcher den im Querschnitt durch die jeweils zugehörige Mittel- bzw. Längsachse weitesten Abschnitt des Kopfkörpers bildet, z.B. mit dem Außenmantel bzw. der Stirnwand einer Kappenform. Der Düsenauslaß ist quer bzw. rechtwinklig zur Mittelachse des Kopfteiles radial nach außen gerichtet und kann in einem radial vorspringenden Bereich des Kopfteiles liegen. Dieser Austragbereich bzw. Austragvorsprung kann unmittelbar durch den Kappenmantel oder durch einen über dessen Außenumfang etwa um das genannte Einführmaß vorstehenden Austragschnabel gebildet sein, dessen Weite im Querschnitt weniger als 15, 10 oder 7 mm beträgt und in dessen freier Endfläche der Düsenauslaß vorgesehen sein kann.

Der Austragkopf kann gemäß der US-A-2 974 880 als Betätigungskopf eine Handhabe zur Betätigung eines Austragförderers, beispielsweise einer Schubkolbenpumpe oder eines druckmittelbeaufschlagten Aerosolbehälters o. dgl. bilden, wobei durch die Druck-Betätigung der dabei mit einer einzigen Hand zu haltenden und zu verkürzenden Austrageinheit das Medium vom Austragförderer unter Druck der Düse zugeführt und in deren Bereich durch Querströmung so verwirbelt wird, daß es sich am Düsenauslaß zerstäubt vom Austragkopf ins Freie ablöst. Die Düse bzw. die zum Düsenauslaß unmittelbar benachbarte Zerstäubungseinrichtung kann einteilig oder mehrteilig ausgebildet sein. Bei mehrteiliger Ausbildung besteht die Gefahr, daß ein Düsenteil, insbesondere der vom Düsenauslaß durchsetzte Düsenteil, sich unter dem Förderdruck auch dann vom übrigen Austragkopf löst, wenn er mit einer Schnappverbindung, durch Preßsitz o. dgl. gehaltert ist.

Ist dieser Düsenteil entgegen Austragrichtung des Düsenauslasses in seine Halterung eingesetzt bzw. etwa in Austragrichtung ohne Verursachung eines Materialbruches lösbar, so kann er unter dem Förderdruck abgesprengt werden und bei Anwendung in einer Körperöffnung zu schweren gesundheitlichen Beeinträchtigungen, insbesondere zum Eindringen in die Luftröhre des Benutzers führen. Ähnliches gilt auch bei einteiliger Ausbildung der Düse bzw. der Leiteinrichtung für das Medium oder dann, wenn der Düsenteil sich durch Bruchbeschädigung teilweise oder ganz vom übrigen Austragkopf lösen kann.

Dies läßt sich verhältnismäßig einfach vermeiden, wenn gemäß der US-A-4 809 083 die Düsenachse und die Betätigungs- bzw. Mittelachse des Kopfteiles parallel zueinander oder achsgleich liegen, weil dann der Austragstutzen mit dem zur Betätigung mit den Fingern erweiterten Kopfteil einteilig ausgebildet werden kann und außerdem der Düsenkern vom stromaufwärts liegenden Ende her in eine Aufnahme eingesetzt werden kann, welche den Medien-Druckraum des Austragförderers unmittelbar und geradlinig mit der Düse verbindet. In dieser Aufnahme liegt der meist sehr enge Düsen-Endkanal, der von der Druckkammer bzw. dem Anschluß eines an diese angeschlossenen, mehrfach weiteren Anschlußkanales bis zur Querumlenkung am inneren Ende des Düsenkanales annähernd geradlinig und/oder mit konstantem Durchflußquerschnitt durchgeht.

Für zahlreiche Anwendungen, insbesondere für die Anwendung tief in Öffnungen, ist es ergonomisch zweckmäßiger, wenn die Düsenachse quer zur Betätigungsachse bzw. zu derjenigen Achse liegt, um welche herum die Austrageinheit beim Austrag mit der Hand umgriffen wird oder wenn gemäß der US-A-5 121 883 die Düsenachse bzw. Düse anders gegenüber dieser Griffachse seitlich versetzt ist. Hier läßt sich die Düse, Dralleinrichtung oder ein Düsenkern bzw. Füllstück durch das in der Betätigungs- bzw. Griffachse liegende Kanalgehäuse hindurch in Austrags-Strömungsrichtung des Mediums montieren oder als Formteil aus Kunststoff o. dgl. herstellen. Gemäß der US-A-3 990 639 ist der vom Düsenauslaß durchsetzte Düsenteil von der Außenseite des Kopfkörpers her, beispielsweise entgegen Austragrichtung, zu montieren. Bei den bekannten Austragköpfen mit außerhalb der Betätigungsachse liegendem Düsenauslass liegt das Verbindungsglied für die Verbindung mit einem Betätigungsstößel des Austragförderers frei. Der Erfindung liegt die Aufgabe zugrunde, einen Austragkopf bzw. eine Austrageinheit der genannten Art zu schaffen, bei welcher Nachteile bekannter Ausbildungen bzw. der beschriebenen Art vermieden sind und die insbesondere dagegen gesichert ist, daß Kopfteile abgesprengt sowie durch die Strömungsenergie des Mediums vom Austragkopf weggeschleudert werden.

Erfindungsgemäß sind die Merkmale nach Patentanspruch 1 vorgesehen. Die Düse ist in den Kopfteil bzw. den Austragvorsprung so integriert, daß das Absprengen von Düsenpartikeln bzw. Düsenteilen vermieden ist, insbesondere das Absprengen durch Versagen einer Halterung für diesen Düsenteil, welche den Düsenteil ohne Materialbruch freigeben kann. Die Sicherung kann durch formschlüssige Verbindung und/oder durch einteilige Ausbildung des Düsenteiles mit dem Kopfteil erreicht werden. Die formschlüssige Sicherung schließt zweckmäßig mindestens einen gegen Bewegungen etwa in Austragrichtung wirksamen Anschlag ein, der beispielsweise durch einen auf den Austragvorsprung erwirkenden Anschlag des Kopfteiles bzw. Kopfgehäuses gebildet sein kann.

Unabhängig von der beschriebenen Ausbildung kann es, insbesondere zur einfachen Fertigung bzw. Montage, vorteilhaft sein, in einem vom Bereich des Austragvorsprunges entfernten Bereich des Kopfteiles eine Zugangsöffnung vorzusehen, aus welcher z.B. ein Formschieber einer Spritzform nach der Herstellung des Kopfkörpers herausgezogen oder ein Bauteil, wie ein Düsenteil, von außen eingesetzt werden kann. Die Zugangsöffnung durchsetzt nicht das Anschlußglied zur Verbindung des Austragkopfes mit der Stößeleinheit bzw. der Druckkammer des Austragförderers, sondern liegt in einer von der Achse dieses Anschlußgliedes abweichenden Achse, beispielsweise achsgleich zum Düsenkanal.

Dadurch kann dieser Düsenkanal in Austragrichtung verengt ausgebildet sein und eine innere Stirnfläche durchsetzen, welche mit vertieften bzw. vorspringenden Leitflächen versehen ist. Für den Gebrauch wird die Zugangsöffnung zweckmäßig mit einem Verschluß, beispielsweise einem eingepreßten oder eingeschnappten Stopfen, dicht verschlossen, während der an die Zugangsöffnung bis zum Düsenauslaß anschließende Aufnahmekanal für die Durchströmung mit dem Medium durchlässig bleibt. Zweckmäßig ist dieser Strömungsweg bzw. der gesamte Austragkopf von druck- und/oder wegabhängig arbeitenden Ventilen frei.

Bevorzugt wird der Kopfkörper vollständig von einem Querkanal durchsetzt, der an einem Ende den Düsenauslaß und am anderen Ende eine verschließbare Zugangsöffnung bildet, wobei zwischen diesen Enden an den Querkanal ein Axialkanal für das Medium anschließt, von welchem das Medium unter winkelförmiger Umlenkung in den Querkanal und von diesem zur Düse gelangt.

Insbesondere bei entsprechend langer Ausbildung des Austragvorsprunges kann in diesen und in den Kopfteil ein Sicherungsanker derart eingreifen, daß selbst beim Abbrechen des Austragvorsprunges der abgebrochene Teil durch den Anker gegenüber dem Kopfteil lagegesichert bleibt und sich nicht vom Kopfteil lösen kann. Dieser Anker kann durch den Düsenkern bzw. das Füllstück gebildet sein, welches zweckmäßig annähernd über die gesamte Länge des zugehörigen Kanales durchgeht und einen Abschnitt der äußersten Außenfläche des Austragkopfes bildet. Der Austragkopf kann aus nur einem einzigen einteiligen Bauteil oder aus nur zwei jeweils einteiligen Bauteilen aus Kunststoff o. dgl. bestehen.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Ausführungsbeispiele der Erfindung sind in den Zeichnungen dargestellt und werden im folgenden näher erläutert. In den Zeichnungen zeigen:

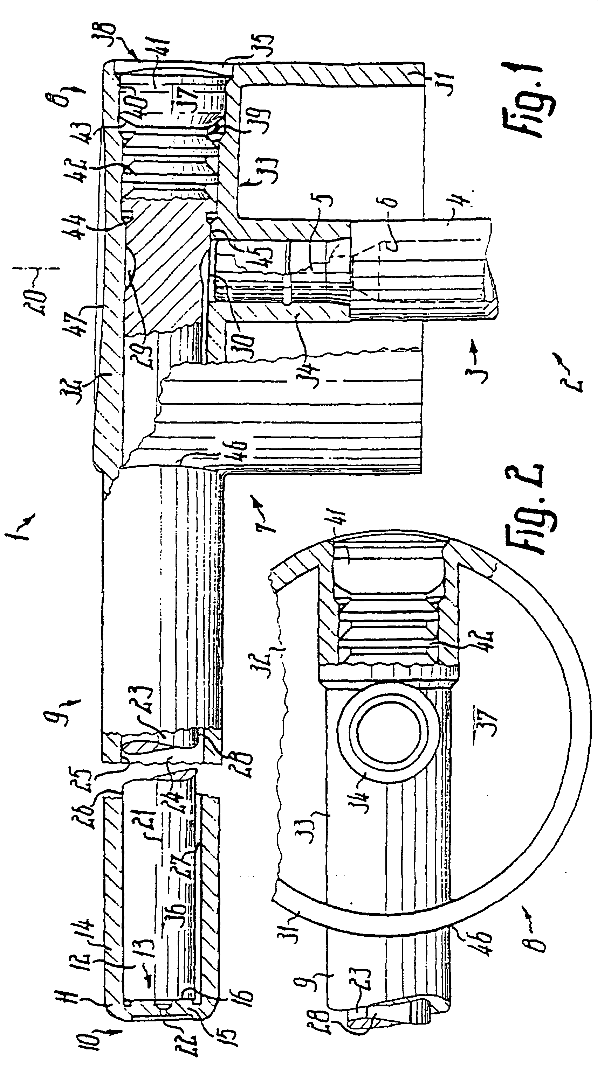

- Fig. 1: einen erfindungsgemäßen Austragkopf in teilweise geschnittener Ansicht,
- Fig. 2: den Austragkopf gemäß Fig. 1 in teilweise geschnittener Ansicht auf die Unterseite,

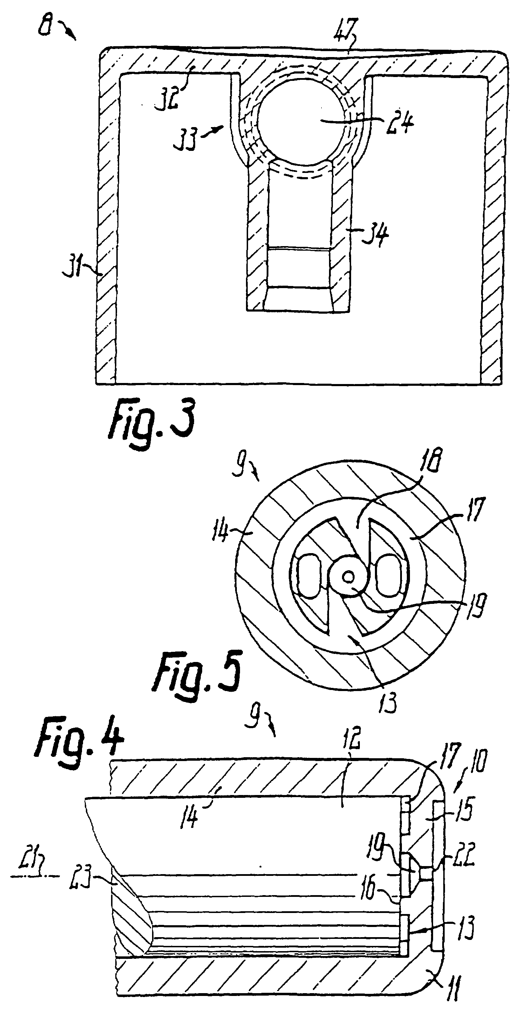

- Fig. 3: einen Schnitt durch den Kopfkörper des Austragkopfes,
- Fig. 4: den Düsenbereich in vergrößerter Darstellung und
- Fig. 5: einen Schnitt durch die Leiteinrichtung des Düsenbereiches gemäß Fig. 4.

Der Austragkopf 1 dient zur Betätigung eines Austragförderers 2, nämlich einer Schubkolbenpumpe, an deren Ende der Austragkopf 1 als Endstück achsgleich angesetzt ist und deren Pumpengehäuse er axial übergreift. Aus dem Pumpengehäuse steht axial eine Betätigungs- bzw. Kolbeneinheit 3 vor, welche innerhalb des Pumpengehäuses einen die Druck- bzw. Pumpkammer durch hin- und hergehendes Verschieben verengenden sowie erweiternden Pumpkolben und ggf. ein öffnendes und schließendes Auslaßventil trägt. Die Kolbeneinheit 3 weist einen in den Austragkopf 1 vorstehenden Betätigungsstößel 4 auf, dessen freies Ende ein verengtes, zapfenförmiges Gegenglied 5 zur Verbindung mit dem Austragkopf 1 bildet. Durch Hineinschieben der Kolbeneinheit 3 in das Pumpengehäuse gelangt das Medium in der Druckkammer unter Druck, wonach das Auslaßventil öffnet und das Medium durch einen Auslaßkanal 6 in der Kolbeneinheit 3 in den Kopf einströmt. Nach Freigabe des Austragkopfes 1 von der Betätigungskraft kehrt er unter der Wirkung einer Rückstellfeder mit der Kolbeneinheit 3 in seine Ausgangslage zurück, wobei das Auslaßventil schließt und ein während des Austrages geschlossenes Einlaßventil öffnet, so daß durch dieses eine weitere Mediendosis in den Druckraum aus einem Medienspeicher angesaugt wird.

Der Austragkopf 1 besteht nur aus zwei einteiligen sowie aus gleichen oder unterschiedlichen Kunststoffen hergestellten Bauteilen, von denen der Kopfkörper 7 zur unmittelbaren Verbindung mit dem Gegenglied 5 dient, während der andere Bauteil gegenüber dem Austragförderer 2 bzw, der Kolbeneinheit 3 berührungsfrei ist und vollständig versenkt innerhalb des Kopfkörpers 7 liegt. Der Kopfkörper 7 weist einen unmittelbar mit dem Austragförderer 2 bzw. der Kolbeneinheit 3 in seinem Innern verbundenen, kappenförmigen Kopfteil 8 auf, welcher den Austragförderer 2 in jeder Stellung am Außenund/oder Innenumfang übergreifen kann und in dem vollständig versenkt das Gegenglied 5 liegt.

Über den Außenumfang des zylindrischen Kopfteiles 8 sowie mit Abstand zwischen dessen stirnseitigen Endflächen steht ein Austragglied 9 bzw. ein Vorsprung des Kopfkörpers 7 um ein Maß vor, das mindestens zwei- bis dreimal größer als die Außenweite des Kopfteiles 8 ist. Diese Außenweite ist mindestens drei- bis viermal größer als die Außenweite des Austraggliedes 9, das als Rachenschnabel zum Einführen in die Mundhöhle des Benutzers dient und wie der Kopfteil 8 über seine gesamte Länge eine glatte, von scharfen Kanten freie Außenfläche mit durchgehend konstanter Außenweite hat. Das Austragglied 9 liegt in einer Axialebene des Kopfteiles 8 und zu diesem rechtwinklig. Die Länge des Austraggliedes 9 ist mindestens drei- bis viermal größer als die des Kopfteiles 8, und demgegenüber ist die Länge des anderen Bauteiles nochmals größer, nämlich etwa um die Außenweite des Kopfteiles 8.

Das freie Ende des Austraggliedes 9 weist eine Düse 10 auf, die nur aus zwei Düsenteilen 11, 12 besteht, von denen der eine einteilig mit dem Kopffeile 8 und dem Austragglied 9 und der andere einteilig mit dem weiteren Bauteil ausgebildet sein kann. Der kappenförmige, am Außenumfang und Ende freiliegende Düsenteil 11 schließt einteilig an das rohrförmige Austraglied 9 an und ist durch einen in der Außenweite kontinuierlich fortgesetzten Endabschnitt dieses Rohres gebildet, so daß die Länge der einteiligen Düsenkappe, nämlich des Düsenteiles 11, mindestens sechs- bis achtmal größer als die Außenweite sein kann.

Der andere Düsenteil 12 ist als Düsenkern vorgesehen, welcher vollständig versenkt sowie in Axialrichtung festsitzend innerhalb des Düsenteiles 11 bzw. des Austraggliedes 9, z.B. mit Preßsitz, angeordnet ist. Mit ihren einander zugekehrten Endflächen bilden die Düsenteile 11, 12 eine Drall- bzw. Leiteinrichtung 13, durch welche das Medium an bzw. zwischen diesen Endflächen von einer axialen Strömungsrichtung quer umgelenkt und dann in eine Wirbel- bzw. Rotationsströmung um die Düsenachse versetzt wird.

Der Kappenmantel 14 der Düse 10, welcher gleichzeitig den Rohrmantel des Gliedes 9 bildet, geht an dessen freien Ende einteilig in eine Kappenstirnwand 15 über, welche mit ihrer Innenseite die eine der beiden genannten Stirnflächen bildet. An dieser Stirnfläche 16 liegt der Düsenteil 12 mit seiner glatten und durchgehend ebenen Endfläche an. Zwischen diesen Endflächen sind Leitkanäle 17 bis 19 vorgesehen, welche ausschließlich durch nutförmige Vertiefungen in der Stirnfläche 16 gebildet sind. An den Außenumfang des Düsenteiles 12 schließt als Leitfläche der Leitkanal 17 als Ringkanal an, von dem zwei gleichmäßig über den Umfang verteilte Leitkanäle 18, nämlich Quer- bzw. Tangentialkanäle, ausgehen. Jeder dieser Leitkanäle 18 mündet tangential in das Innere, weiteste Ende des als Düsenkanal vorgesehenen Leitkanales 19, der von der Stirnfläche 16 ausgehend die Kappenstirnwand 15 in der Düsenachse 21 durchsetzt. Die Mittel- bzw. Düsenachse 21 liegt in der Mittelachse des Austraggliedes 9 und rechtwinklig zur Mittelachse bzw. Betätigungsachse 20 des Kopfteiles 8. Der Leitkanal 19 ist zur Außenseite der Kappenstirnwand 15 verengt und bildet in dieser Außenseite den Düsenauslaß 22, an welchem das Medium beim Austrag von dem Austragkopf 1 freikommt. Diese Düsenöffnung hat eine Weite von weniger als einem oder einem halben Millimeter und kann in der Außenseite vertieft liegen.

Der zweite Bauteil des Austragkopfes 1 ist ein durchgehend geradliniges, stabförmiges und bis auf eine Abflachung achssymmetrisches Füllstück 23 für den Ropfteil 8 und das Austragglied 9. Das Füllstück 23 kann an einem Ende einteilig mit dem Düsenteil 12 ausgebildet sein bzw. ist wie dieser festsitzend in einer Aufnahme 24 angeordnet. Diese Aufnahme 24 ist vom Innenumfang 25 des Kappenmantels 14 begrenzt, an welchem der Düsenteil 12 über den größten Teil der Länge bzw. der gesamten Länge des Austraggliedes 9 mit Radialpressung kraftschlüssig mit seinem Außenumfang 26 festsitzt.

Der Außenumfang des Düsenteiles 12 und des Füllstückes 23 ist von der Leiteinrichtung 13 bis zum Kanalübergang zum Auslaßkanal 6 mit einer einzigen Abflachung 28 versehen, welche sich über einen Bogenwinkel von weniger als 90 bzw. 45° erstreckt und mit dem im Querschnitt durchgehend kreisrunden Innenumfang 25 einen Endkanal 27 begrenzt. Der Endkanal 27 hat wie die Leitkanäle 17 bis 19 den geringen Durchflußquerschnitt von Kapiallarkanälen und gegenüber den Leitkanälen 17 bis 19 einen etwas größeren sowie über seine Länge durchgehend konstanten Durchflußquerschnitt, gegenüber welchem der größte bzw. kleinste Durchflußquerschnitt des Auslasskanales 6 mindestens zwei- bis dreifach oder auch mindestens vierfach größer ist.

Das hintere Ende des Auslaßkanales 27 bzw. der Abflachung 28 schließt an eine ringförmig von einem Innen- und Außenumfang begrenzte Kammer, wie eine Verteilkammer 29, an. Diese Kammer ist durch eine im Querschnitt teilkreisförmig vertiefte Nut im Außenumfang des Füllstückes 23 gebildet und ihre Mittelebene liegt etwa in einer Axialebene der Betätigungsachse 20 des Auslasskanales 6. Ein begrenzter Umfangsbereich der Verteilkammer 29 liegt dem verengten Endabschnitt des Auslasskanales 6 mit geringem Abstand unmittelbar gegenüber, so dass ein Kanalübergang 30 gebildet ist, in welchem das Medium aus dem Auslasskanal 6 radial gegen die Bodenfläche der Verteilkammer 29 in diese strömt sowie dabei in eine Ringströmung übergeht. Aus der Verteilkammer 29 fließt das Medium dann unter Strömungsbeschleunigung unmittelbar in das hintere Ende des Endkanales 27. Der Endkanal 27 bzw. der aus Düsenteil 12 und Füllstück 23 bestehende Kern kann so um die Düsenachse 21 gegenüber dem Kopfkörper 7 in jeder beliebigen Drehlage mit gleicher Wirkung liegen, so daß die automatische Montage des Füllstückes 23 ohne die Notwendigkeit einer Drehausrichtung erleichtert ist.

Der Kopfteil 8 weist in der Betätigungsachse 20 einen äußersten Kappenmantel 31 und an dessen näher beim Austragglied 9 liegenden Ende eine äußerste Kappenstirnwand 32 auf, über deren Außenseite der Kappenmantel 31 nicht vorsteht. Benachbart zur Innenseite der Kappenstirnwand 32 bzw. einteilig an sie anschließend ist im Innern des Kappenmantels 31 ein Kanalgehäuse 33 vorgesehen, das sich nur über einen kleineren Teil der Länge des Kappenmantels 31 erstreckt, eine innere Fortsetzung des Kappenmantels 31 bildet und nur mit seinen Enden an den Innenumfang des Kappenmantels 31 anschließt. Zwischen diesen Enden liegt das Kanalgehäuse 33 mit seitlichem Abstand von diesem Innenumfang, so daß es eine U-Form bildet, die mit den Schenkeln einteilig in die Innenseite der Kappenstirnwand 32 übergeht.

Das Kanalgehäuse 33 des Kopfkörpers 7 weist in seinem von der Kappenstirnwand 32 abgekehrten Mantelbereich ein hülsenförmiges Verbindungsglied 34 auf, das in der Betätigungsachse 20 von der Kappenstirnwand 32 weggerichtet frei vom Kanalgehäuse 33 absteht, gegenüber der zugehörigen offenen Stirnfläche des Kappenmantels 31 bzw. des Kopfteiles 8 zurücksteht und zur festsitzenden Verbindung mit dem Gegenglied 5 vorgesehen ist. Das Gegenglied 5 liegt mit Pressung und/oder Schnappsitz innerhalb des Verbindungsgliedes 34, das sich mit seiner Endfläche an einer Übergangsschulter zwischen Gegenglied 5 und übrigem Betätigungsstößel 4 durch Anschlag abstützt. Die Endfläche des Gegengliedes 5 mit der Austrittsöffnung des Auslaßkanales 6 liegt dann annähernd im Innenumfang des Kanalgehäuses, das eine Fortsetzung der Aufnahme 24 bildet. Das Verbindungsglied 34 liegt etwa in der Mitte der Länge des Kanalgehäuses 33.

Das Medium tritt aus dem Düsenauslaß 22 axial in Austragrichtung 36 aus, und auch der Kern aus Düsenteil 12 und Füllstück 23 ist bei der Montage in dieser Richtung, nämlich in Montagerichtung 37, in den Kopfkörper 7 eingesetzt, nämlich quer bzw. rechtwinklig zur Betätigungsachse 20 und parallel zur Düsenachse 21. Hierzu bildendie Aufnahme 24 bzw. das Kanalgehäuse 33 und der Kopfteil 8 in dem vom Austragglied 9 abgekehrten äußersten Bereich eine gegenüber der Aufnahme 24 erweiterte Montageöffnung 35, die bei bzw. nach der Montage mit einem Verschluß 38 so dicht verschlossen wird, dass dessen Verschlußteil 39 mit einer Stirnfläche einen ballig gekrümmten Abschnitt des Außenumfanges des Kopfteiles 8 bildet. Der Verschlußteil 39 ist gegenüber dem Kopfteil 8 durch eine ausschließlich innen liegende, axial zusammenzufügende Verbindung 40 festgesetzt, welche eine Schweiß-, Klebe-, Rast-, Anschlag-, Preß- und/oder Schnapp-Verbindung sein kann. Das hinterste Ende des Füllstückes 23 weist einen gegenüber dessen übrigen Bereichen erweiterten Kopf 41 auf, welcher über die Schnappverbindung am Innenumfang des Kanalgehäuses 33 gesichert ist.

Nach vorne schließt sich an den Kopf 41 unmittelbar eine Mehrzahl von axial benachbarten, ringförmigen Krall- und Dichtgliedern 42 an, die nach Art von Dichtlippen an den Innenumfang des Kanalgehäuses 33 anschließen. Die vordere Stirnfläche des Kopfes 41 bildet einen ringförmigen Anschlag 43, welchem als Gegenanschlag eine ringförmige Innenschulter des Innenumfanges des Kanalgehäuses 33 mit Abstand gegenüberliegt. Dem vordersten Dichtglied 42 liegt ein weiterer Gegenanschlag 44 in entsprechender Weise gegenüber, so dass die Außenweite der Dichtglieder 42 größer als die des Füllstückes 23 und kleiner als die des Kopfes 41 ist. Unmittelbar benachbart zur Verteilkammer 29 und an diese anschließend greift der Außenumfang des Füllstückes 23 über einen Dichtsitz 45 in den Innenumfang 25 so ein, daß der Außenumfang dieses Dichtsitzes 45 an den Innenumfang des Verbindungsgliedes 34 anschließt bzw. über diesen Innenumfang hineinragt. Das aus dem Auslasskanal 6 in den Kanalübergang 30 strömende Medium kann daher nur nach vorne in den Endkanal 27 und nicht nach hinten durch den Verschluß 38 strömen. Die Düse 10 kann statt als Zerstäuber-Düse auch als nicht zerstäubende Düse, z.B. zur Abgabe von Tropfen oder eines unzerstäubten Strahles ausgebildet sein.

Selbst bei Bruch des Kappenmantels 14 bzw. der Kappenstirnwand 15 könnte der Düsenteil 12 bzw. das Füllstück 23 nicht in Austragrichtung 36 abgesprengt werden. Auch weitere Teile des Austraggliedes 9, z.B. der Kappenmantel 14, können nicht in dieser Richtung abgesprengt werden, weil der Düsenteil 12 bzw. das Füllstück 23 über eine so große Länge und Fläche mit dem Rohrteil bzw. Austragglied 9 kraftschlüssig verbunden ist, daß die Haltekraft nicht überwunden werden kann. Außerdem schließt der Rohrteil im Bereich seines Anschlusses 46 einteilig an den Außenumfang bzw. ausschließlich an den Kappenmantel 31 bzw. das Ende des Kanalgehäuses 33 des Kopfteiles 8 an, wie auch an die Kappenstirnwand 15, so dass es dazwischen durchgehend einteilig ist. Die äußere Stirnfläche der Kappenstirnwand 32 sowie ggf. der anschließende Bereich des Außenumfanges des Austraggliedes 9 bildet eine kuhlenförmig vertiefte Handhabe 47 in Form einer Finger-Druckfläche zur Betätigung des Austragkopfes 1 gegenüber dem Grundkörper bzw. Gehäuse des Austragförderers 2 in der Richtung, in welcher der Kappenmantel 31 und das Verbindungsglied 34 frei vorstehen.

Alle angegebenen Eigenschaften und Wirkungen können genau wie beschrieben, wenigstens teilweise oder nur etwa bzw. im wesentlichen wie beschrieben vorgesehen sein, je nachdem für welche Anwendungen der Schnabel- oder Austragkopfkopf 1 vorgesehen ist.

## Patentansprüche

1. Austragkopf für Medien, insbesondere zur medikamentösen Behandlung des Rachens, mit einem Kopfkörper (7), der einen eine Betätigungsachse (20) bestimmenden Kopfteil (8) und ein Austragglied (9) umfaßt, an welchem in einem Düsenbereich eine mindestens einen Düsenteil (11, 12) sowie einen Düsenauslaß (22) aufweisende Düse (10) angeordnet ist, die eine Düsenachse (21) und deren Düsenauslaß (22) eine Austragrichtung (36) bestimmt, wobei die Betätigungsachse (20) eine im wesentlichen von der Düsenachse (21) abweichende Lage einnimmt und der Kopfteil (8) ein zur Betätigunsachse (20) etwa paralleles Verbindungsglied (34) zur Verbindung des Austragkopfes (1) mit einem Betätigungsstößel (4) aufweist, **dadurch gekennzeichnet, dass** der Kopfteil (8) einen äußersten Kappenmantel (31) und ein an dessen Innenumfang anschließendes Kanalgehäuse (33) aufweist, an welches das Verbindungsglied (34) anschließt.

2. Austragkopf nach Anspruch 1, **dadurch gekennzeichnet, daß** ein an die Düse (10) anschließender Endkanal (27) für das Medium von mindestens einem Düsenteil (11, 12) begrenzt ist und ein innerer Düsenteil (12) im Bereich der Düse (10) eine ebene Endfläche aufweist, dass insbesondere der Endkanal (27) im wesentlichen durchgehend wesentlich enger als ein Auslaßkanal (6) ist, der etwa in der Betätigungsachse stromaufwärts an den Endkanal (27) mit einem Kanalübergang (30) anschließt, und dass vorzugsweise die Düse (10) in das Austragglied (9) integriert und insbesondere wenigstens gegen bruchfreies Abläsen von Düsenteilen in Austragrichtung (36) gesichert ist.

3. Austragkopf nach Anspruch 1 oder 2, **dadurch gekennzeichnet, dass** die Düse (10) mindestens zwei gesonderte Düsenteile (11, 12), wie eine Düsenkappe und einen in dieser liegenden Düsenkern enthält, dass insbesondere mindestens einer der Düsenteile (11, 12) einstückig bzw. gegen Ablösebewegungen in Austragrichtung (36) formschlüssig mit dem Kopfkörper (7) verbunden ist, und dass vorzugsweise die Düsenkappe durch einen vorderen Endabschnitt des im wesentlichen durchgehend rohrförmigen Austraggliedes (9) gebildet ist, in dem der Düsenkern hinter einer von dem Düsenauslass (22) durchsetzten Kappenstirnwand (15) der Düsenkappe festsitzend angeordnet ist.

4. Austragkopf nach einerm der vorhergehenden Ansprüche, **dadurch gekennzeichnet, daß** Mittel zum Einsetzen mindestens eines vom Kopfkörper (7) gesonderten Düsenteiles (12) in den Kopfkörper (7) gegen einen Anschlag (16,40,44) in einer Montagerichtung (37) vorgesehen sind, die von einer Gegenrichtung abweicht, welche der Austragrichtung (36) entgegengesetzt ist, dass insbesondere die Montagerichtung (37) zur Austragrichtung (36) im wesentlichen gleichgerichtet ist, und dass vorzugsweise der Kopfkörper (7) für den gesonderten Düsenteil (12) eine Montageöffnung (35) aufweist, die den Kopfkörper (7) im Abstand und gesondert vom Düsenbereich durchsetzt und insbesondere in einem vom Austragglied (9) abgekehrten Bereich des Kopfkörpers (7) liegt.

5. Austragkopf nach einem der vorhergehenden Ansprüche, **dadurch gekennzeichnet, dass** ein Düsenteil (12) durch einen Endabschnitt eines stabförmigen Füllstückes (23) gebildet ist, das in einer Aufnahme (24) des Kopfkörpers (7) liegend den Endkanal (27) annähernd über dessen gesamte Länge begrenzt sowie insbesondere in einem von der Düse (10) entfernten oder zum Kanalübergang (30) benachbarten Bereich einen dichten Permanent-Verschluß (38) der Aufnahme (24) und des Endkanales (27) bildet.

6. Austragkopf nach einem der vorhergehenden Ansprüche, **dadurch gekennzeichnet, daß** mindestens ein den Endkanal (27) begrenzender Düsenteil (11, 12) mit einer hinteren Verlängerung im Bereich des Kanalüberganges (30) eine in unterschiedlichen Drehlagen des Düsenteiles (12) gegenüber der Düsenachse (21) an den Auslasskanal (6) anschließbare sowie an den Endkanal (27) anschließende Verteilkammer (29) begrenzt und daß insbesondere der Endkanal (27) von einer Abflachung (28) am Außenumfang des Düsenteiles (12) begrenzt ist sowie im Querschnitt nur über einen begrenzten Umfangsbereich des Düsenteiles (12) reicht.

7. Austragkopf nach einem der vorhergehenden Ansprüche, **dadurch gekennzeichnet, dass** der Kopfkörper (7) als Betätigungs-Handhabe kappenförmig mit dem äußersten Kappenmantel (31) sowie einer Kappenstirnwand (32) ausgebildet ist, dass insbesondere anschließend an die Innenseite der Kappenstirnwand (32) das in das über den Kappenmantel (31) nach außen vorstehende Austragglied (9) fortgesetzte Kanalgehäuse (33) für den Endkanal (27) vorgesehen ist, und dass vorzugsweise der gesonderte Düsenteil (12) von der vom Austragglied (9) abgekehrten Seite des Kappenmantels (31) des Kopfteiles (8) in das Kanalgehäuse (33) eingesetzt ist.

8. Austragkopf nach einem der vorhergehenden Ansprüche, **dadurch gekennzeichnet, dass** insbesondere das Verbindungsglied (34) als frei abstehender Rohrstutzen an das nur mit seinen Enden mit dem Kappenmantel (31) verbundene Kanalgehäuse (33) quer anschließt, und dass vorzugsweise das Innere des Verbindungsgliedes (34) gegen die Verteilkammer (29) gerichtet ist.

9. Austragkopf nach einem der vorhergehenden Ansprüche, **dadurch gekennzeichnet, dass** das Austragglied (9) mit einem der Düsenteile (11, 12) einteilig ausgebildet ist, dass insbesondere das Austragglied (9) einteilig mit dem Kopfteil (8) ausgebildet ist, und dass vorzugsweise der Austragkopf (1) aus nur zwei jeweils einteiligen Bauteilen, nämlich dem Kopfkörper (7) und einem Kernkörper, zusammengesetzt ist.

10. Austragkopf nach einem der vorhergehenden Ansprüche, **dadurch gekennzeichnet, dass** dem Düsenauslass (22) eine das Medium quer nach innen gegen die Düsenachse (21) umlenkende Leiteinrichtung (13), wie eine Dralleinrichtung, unmittelbar vorgeschaltet ist, dass insbesondere nutförmige, von zwei Stirnflächen (16) begrenzte Leitkanäle (17 bis 19) in einer inneren Stirnfläche (16) des Austraggliedes (9) vorgesehen sind, und dass vorzugsweise der Düsenkern (12) einen außerhalb des Austraggliedes (9) im Kopfkörper (7) durch Anschlag zuggesicherten sowie klemmend in das Austragglied (9) eingreifenden Halteanker für das Austragglied (9) bildet, das als Rachenschnabel zum Einführen in die Mundhöhle sowie bis in den Rachenbereich ausgebildet ist.

11. Austragkopf nach einem der Ansprüche 3 bis 10, **dadurch gekennzeichnet, dass** an die Kappenstirnwand (15) der Düsenkappe stromaufwärts ein rohrförmiger Kappenmantel (14) anschließt, der einteilig mit der Kappenstirnwand (15) ausgebildet ist, dass insbesondere das in der Austragrichtung (36) in die Düsenkappe eingeführte Füllstück (23) mit seinem hinteren Ende außerhalb der Düsenkappe (11) mit einer Preß-Verbindung (40) dicht in eine Montageöffnung (35) eingesetzt und eine Umfangsfläche (28) des Füllstückes (23) als Begrenzung des einzigen Endkanales (27) vorgesehen ist, und dass vorzugsweise das hintere Ende des vom Füllstück (23) begrenzten Endkanales (27) an die ringförmige, von einem Innen- und einem Außenumfang begrenzte Verteilkammer (29) anschließt, welche im wesentlichen in einer Axialebene des Verbindungsgliedes (34) liegt, wobei Mittel zum strömungsbeschleunigten Übertritt des Mediums aus der Verteilkammer (29) in das hintere Ende des Endkanales (27) vorgesehen sind.

## Claims

1. A discharge head for media, more particularly, for the medicinal treatment of the throat, including a head body (7), comprising a head part (8) defining an actuating axis (20) and a discharge member (9) at which in a nozzle portion a nozzle (10) comprising at least one nozzle part (11, 12) as well as a nozzle orifice (22) is arranged, said nozzle (10) defining a nozzle axis (21) and said nozzle orifice (22) thereof a discharge direction (36), whereby said actuating axis (20) assumes a position substantially differing from said nozzle axis (21) and said head part (8) comprises a connecting member (34) roughly parallel to said actuating axis (20) for connecting said discharge head (1) to an actuating plunger (4), **characterized in that** said head part (8) comprises an outermost cap shell (31) and a passage housing (33) adjoining said inner circumference of said latter, said connecting member (34) adjoining said passage housing (33).

2. The discharge head as set forth in claim 1, **characterized in that** an end passage (27) adjoining said nozzle (10) for the medium is defined by at least one nozzle part (11, 12) and an inner nozzle part (12) in the region of said nozzle (10) comprises a flat end surface area, more particularly said end passage (27) being substantially narrower throughout than an outlet passage (6) and adjoining roughly in said actuating axis said end passage (27) with a passage transition (30), and preferably said nozzle (10) being integrated in said discharge member (9) and more particularly is safeguarded at least against fracture release of said nozzle parts in said discharge direction (36).

3. The discharge head as set forth in claim 1 or 2, **characterized in that** said nozzle (10) contains at least two separate nozzle parts (11, 12) such as a nozzle cap and a nozzle core located therein, more particularly at least one of said nozzle parts (11, 12) being connected integrally or positively to said head body (7) in preventing release movements in said discharge direction (36), and preferably said nozzle cap being formed by a proximal end section of said discharge member (9) configured substantially tubular throughout, in which said nozzle core is fixedly arranged behind a cap face wall (15) of said nozzle cap penetrated by said nozzle orifice (22).

4. The discharge head as set forth in any of the preceding claims, **characterized in that** means are provided for inserting at least one nozzle portion separate from said head body (7) into said head body (7) against a stop (16, 40, 44) in an assembly direction (37) differing from a counter-direction opposite to said discharge direction (36), more particularly said assembly direction (37) being oriented substantially identical to said discharge direction (36), and preferably said head body (7) comprising for said separate nozzle part (12) an assembly opening (35) passing through said head body (7) spaced away and separate from said nozzle portion in particularly being located in a portion of said head body (7) facing away from said discharge member (9).

5. The discharge head as set forth in any of the preceding claims, **characterized in that** said nozzle part (12) is formed by an end section of a rod-shaped filler (23) which located in a mount (24) for said head body (7) defines said end passage (27) more or less over the full length thereof and, more particularly, forming in a portion remote from said nozzle (10) or adjacent to said passage transition (30) a sealing permanent closure (38) of said mount (24) and of said end passage (27).

6. The discharge head as set forth in any of the preceding claims, **characterized in that** at least one nozzle part (11, 12) defining said end passage (27) with a distal elongation in the region of the passage transition (30) defines a distribution chamber (29) connectable to said outlet passage (6) in differing rotary positions of said nozzle part (12) with respect to said nozzle axis (21) to outlet passage (6) as well as adjoining said end passage (27) and, more particularly, said end passage (27) being defined by a flat (28) on the outer circumference of said nozzle part (12) and extending in cross-section merely over a limited circumferential portion of said nozzle part (12).

7. The discharge head as set forth in any of the preceding claims, **characterized in that** said head body (7) is configured cap-shaped as an actuating finger-grip with an outermost cap shell (31) as well as a cap face end wall (32), more particularly, approximately adjoining the inner side of said cap face end wall (32) a passage housing (33) being provided for said end passage (27), said housing (33) projecting into said discharge member (9) protruding outwardly beyond said cap shell (31), and preferably said separate nozzle part (12) being inserted into said passage housing (33) from the side of said cap shell (31) lot said head part (8) facing away from said discharge member (9).

8. The discharge head as set forth in any of the preceding claims, **characterized in that** more particularly, said connecting member (34) transversally adjoins as a freely protruding tubular port said passage housing (33) connected by its ends to said cap shell (31), and preferably the interior of said connecting member (34) being oriented against said distribution chamber (29).

9. The discharge head as set forth in any of the preceding claims, **characterized in that** discharge member (9) is configured integral with one of said nozzle parts (11, 12), more particularly, said discharge member (9) being configured integral to said head part (8) and preferably said discharge head (1) being composed of only two integral components in each case, namely said head body (7) and a core body.

10. The discharge head as set forth in any of the preceding claims, wherein a guiding means (13), such as a swirler, diverting the medium transversely inwards against the nozzle axis (21) is directly inserted upstream of said nozzle outlet (22), more particularly, groove-type guide passages (17 to 19) defined by two face end surface areas (16) being provided in an inner face end surface area (16) of said discharge member (9) and preferably said nozzle core (12) forming a retaining anchor for said discharge member (9) outside of said discharge member (9) in said head body (7) tensionally locked in place by an abutment as well as clampingly engaging said discharge member (9) which is configured as a throat beak for introduction into the mouth cavity and up to the region of the throat.

11. The discharge head as set forth in any of the claims 3 to 10, **characterized in that** a tubular cap shell (14) integrally configured with said cap face wall (15) adjoins upstream said cap face wall (15) of said nozzle cap, more particularly said filler (23) inserted in said discharge direction (36) into said nozzle cap being inserted by its distal end outside of said nozzle cap (11) with a press-fit connection (40) sealingly into an assembly opening (35) and a circumferential surface area (28) of said filler (23) being provided as a definition of said sole end passage (27), and preferably said distal end of said end passage (27) defined by said filler (23) adjoining said tubular distribution chamber (29) defined by an inner and an outer circumference, said distribution chamber (29) being located substantially in an axial plane of said connecting member (34), whereby means are provided for the flow-accelerated transition of the medium from said distribution chamber (29) into the distal end of said end passage (27).

## Revendications

1. Tête de décharge pour des matières, notamment pour le traitement médicamenteux du pharynx, avec un corps de tête (7) comprenant une pièce de tête (8) et un élément de décharge (9), auprès duquel est disposé dans le domaine de buse une buse (10), présentant au moins un élément de buse (11, 12) ainsi qu'une sortie de buse (22), qui définit un axe de buse (21) et sa sortie de buse (22) définissant un sens de décharge (36), l'axe d'actionnement (20) adoptant une position essentiellement différente de l'axe de buse (21) et la pièce de tête (8) présentant un élément de raccordement (34) à peu près parallèle à l'axe d'actionnement (20) pour le raccordement de la tête de décharge (1) avec un coulisseau d'actionnement (4), **caractérisée en ce que** la pièce de tête (8) présente une enveloppe de capuchon (31) extérieure et un boîtier de canal (33) faisant suite à la circonférence intérieure de celle-ci, auquel suit l'élément de raccordement (34).

2. Tête de décharge d'après la revendication 1, **caractérisée en ce qu'**un canal terminal (27) à la suite de la buse (10) est délimité pour la matière au moins d'un élément de buse (11, 12) et qu'un élément de buse (12) intérieur présente une surface terminale plane dans le domaine de la buse (10), **en ce que** notamment le canal terminal (27) est beaucoup plus étroit en substance de manière continue qu'un canal d'évacuation (6), qui suit en amont à peu près dans l'axe d'actionnement le canal terminal (27) par un raccord de canaux (30), et **en ce que** de préférence la buse (10) est intégrée dans l'élément de décharge (9) et qu'elle est notamment assurée au moins contre un détachement de pièces de buse en direction de décharge (36).

3. Tête de décharge d'après la revendication 1 ou 2, **caractérisée en ce que** la buse (10) comprend au moins deux éléments de buse (11, 12) individuels, tel qu'un capuchon de buse et un noyau de buse situé dans celui-ci, **en ce que** notamment au moins un des éléments de buse (11, 12) est raccordé d'une seule pièce ou encore à engagement positif contre des mouvements de détachement en direction de décharge (36) avec le corps de tête (7), et **en ce que** de préférence le capuchon de buse est formé par une partie terminale antérieure de l'élément de décharge (9) tubulaire et essentiellement continu, dans lequel est disposé de manière fixe le noyau de buse derrière une paroi frontale de capuchon (15) du capuchon de buse, traversée par l'orifice de buse (22).

4. Tête de décharge d'après une des revendications précédentes, **caractérisée en ce que** des moyens sont prévus pour l'insertion au moins d'un élément de buse (12), séparé du corps de tête (7), dans le corps de tête (7) contre un arrêt (16, 40, 44) dans une direction de montage (37) différente par rapport à un sens contraire opposé à la direction de décharge (36), et **en ce que** notamment la direction de montage (37) est essentiellement orientée dans la même direction par rapport à la direction de décharge (36), et **en ce que** de préférence le corps de tête (7) présente pour l'élément de buse (12) individuel une ouverture de montage (35), qui traverse le corps de tête (7) séparément et à une certaine distance du domaine de buse et qui se trouve notamment dans un domaine du corps de tête (7) orienté en direction contraire par rapport à l'élément de décharge (9).

5. Tête de décharge d'après une des revendications précédentes, **caractérisée en ce qu'**un élément de buse (12) est formé par une section terminale d'une pièce de remplissage (23) en forme de tige, laquelle pièce délimite, en se trouvant dans un logement (24) du corps de tête (7), le canal terminal (27) quasiment sur toute sa longueur ainsi qu'elle forme notamment dans un domaine éloigné de la buse (10) ou bien limitrophe au raccord de canaux (30), une occlusion permanente (38) du logement (24) et du canal terminal (27).

6. Tête de décharge d'après une des revendications précédentes, **caractérisée en ce qu'**au moins un des éléments de buse (11, 12), qui délimite le canal terminal (27), délimite par une rallonge postérieure dans le domaine du raccord de canaux (30) une chambre de distribution (29), pouvant être raccordée au canal de sortie (6) dans des positions angulaires différentes de l'élément de buse (12) par rapport à l'axe de buse (21) ainsi qu'elle fait suite au canal terminal (27) et **en ce que** notamment le canal terminal (27) est délimité par un aplatissement (28) à la circonférence extérieure de l'élément de buse (12) ainsi qu'il s'étend en section transversale seulement sur une partie limitée du domaine de circonférence de l'élément de buse (12).

7. Tête de décharge d'après une des revendications précédentes, **caractérisée en ce que** le corps de tête (7) en tant que manette d'actionnement est réalisée en forme de capuchon avec l'enveloppe de capuchon (31) la plus extérieure ainsi qu'avec une paroi frontale de capuchon (32), **en ce que** le boîtier de canal (33) pour le canal terminal (27), prolongé dans l'élément de décharge (9) saillant au-dessus de l'enveloppe de capuchon (31) vers l'extérieur est prévu notamment à la suite de la face intérieure de la paroi frontale de capuchon (32), et **en ce que** de préférence l'élément de buse (12) individuel est inséré dans le boîtier de canal (33) par le côté de l'enveloppe de capuchon (31) de la pièce de tête (8), opposé à l'élément de décharge (9).

8. Tête de décharge d'après une des revendications précédentes, **caractérisée en ce que** notamment l'élément de raccordement (34) fait suite transversalement, en tant que tubulure librement saillante, au boîtier de canal (33), qui est raccordé uniquement par ses extrémités à l'enveloppe de capuchon (31), et **en ce que** de préférence l'intérieur de l'élément de raccordement (34) est orienté vers la chambre de distribution (29).

9. Tête de décharge d'après une des revendications précédentes, **caractérisée en ce que** l'élément de décharge (9) est réalisé d'une seule pièce avec un des éléments de buse (11, 12), **en ce que** notamment l'élément de décharge (9) est réalisé d'une seule pièce avec la pièce en tête (8), et **en ce que** de préférence la tête de décharge (1) est assemblée à partir de deux éléments constructifs seulement respectivement composés d'une seule pièce, c'est-à-dire du corps de tête (7) et d'un corps de noyau.

10. Tête de décharge d'après une des revendications précédentes, **caractérisée en ce qu'**un moyen de guidage (13) tel qu'un moyen giratoire, déviant la matière transversalement vers l'intérieur vers l'axe de buse (21), est directement mis en précascade sur la sortie de buse (22), **en ce que** notamment des canaux de guidage (de 17 à 19) en forme d'encoche et délimités par deux surfaces frontales (16) sont prévus dans une surface frontale (16) intérieure de l'élément de décharge (9) et **en ce que** de préférence le noyau de buse (12) forme une ancre de retenue pour l'élément de décharge (9), qui est assuré contre traction par arrêt à l'extérieur de l'élément de décharge (9) dans le corps de tête (7) ainsi qu'il s'engage par serrage dans l'élément de décharge (9), lequel est réalisé en tant que bec à introduire dans la cavité buccale ainsi que jusque dans le domaine du pharynx.

11. Tête de décharge d'après une des revendications de 3 à 10, **caractérisée en ce qu'**une enveloppe de capuchon (14) tubulaire fait suite en amont à la paroi frontale de capuchon (15) du capuchon de buse, enveloppe qui est réalisée d'une seule pièce avec la paroi frontale de capuchon (15), **en ce que** notamment la pièce de remplissage (23) introduite en direction de décharge (36) dans le capuchon de buse, est introduite de manière étanche avec son extrémité postérieure à l'extérieur du capuchon de buse (11) avec un emmanchement (40) dans une ouverture de montage (35) et que une surface circonférencielle (28) de la pièce de remplissage (23) est prévue en tant que délimitation de l'unique canal terminal (27), et **en ce que** de préférence l'extrémité postérieure du canal terminal (27) délimité par la pièce de remplissage (23) fait suite à la chambre de distribution (29) annulaire et délimitée par une circonférence intérieure et une circonférence extérieure, laquelle chambre se trouve essentiellement dans un plan axial de l'élément de raccordement (34), des moyens étant prévus pour le passage à écoulement accéléré de la matière hors de la chambre de distribution (29) dans l'extrémité postérieure du canal terminal (27).
